# DEMANDE DE BREVET EUROPEEN

(11) **EP 0 714 983 A1**
(43) Date de publication de la demande: **05.06.1996**
(21) Numéro de dépôt: 95202831.4
(22) Date de dépôt: 19.10.1995
(51) Int. Cl.: C12P 7/64

(54) **Procédé d'hydrolyse des triglycérides d'acides gras polyinsaturés**

(30) Priorité: 28.11.1994 EP 94118663
(71) Demandeur: SOCIETE DES PRODUITS NESTLE S.A., CH-1800 Vevey (CH)
(72) Inventeur: Wille, Hans-Juergen, CH-1844 Villeneuve (CH)
(74) Mandataire: Archambault, Jean

(57) **Abrégé**

Pour préparer un mélange d'acides gras polyinsaturés de qualité optimale, on hydrolyse une huile riche en acides gras polyinsaturés par une lipase non stéréospécifique de Candida cylindracea en milieu émulsionné huile-dans-l'eau en présence de lécithine à titre d'agent émulsionnant et dans des conditions ménageantes, de préférence à la température ambiante et en milieu aqueux tampon de pH proche de la neutralité.

## Description

L'invention concerne l'hydrolyse enzymatique totale des huiles riches en acides gras polyinsaturés.

Les acides gras des séries n-6 et n-3 présentent un intérêt nutritionnel, en particulier en temps que précurseurs de la biosynthèse des prostaglandines. Il peut être avantageux de disposer de fractions enrichies en ces acides gras pour différérentes applications nutritionnelles et cosmétiques. On trouve ces acides gras dans la nature principalement sous forme de triglycérides.

On obtient industriellement les acides gras libres à partir des triglycérides par hydrolyse à haute température et sous forte pression. On peut également obtenir les acides gras libres à partir des triglycérides par voie chimique par saponification avec une base forte, puis libération des acides gras par neutralisation des savons a l'aide d'un acide fort et enfin extraction des acides gras du milieu à l'aide d'un solvant non polaire, par exemple l'hexane.

L'application de ces méthodes peut entraîner une dégradation dans le cas des acides gras polyinsaturés.

Les méthodes enzymatiques représentent une alternative aux méthodes précédentes puisque qu'elles permettent d'effectuer les réactions dans des conditions douces en utilisant peu d'énergie et un équipement moins sollicité.

On conduit habituellement l'hydrolyse en milieu biphasique en maintenant une agitation efficace pour assurer le meilleur contact possible entre la phase aqueuse et la phase organique.

Nous avons trouvé une méthode enzymatique simple d'hydrolyse totale des triglycérides d'une huile qui permet d'améliorer de manière inattendue l'hydrolyse en obtenant un taux de conversion en acides gras supérieur au taux obtenu par la méthode enzymatique en milieu biphasique et ceci dans des conditions peu dégradantes qui permettent une amélioration de la qualité et de la stabilité des acides gras polyinsaturés par rapport à la voie chimique.

L'invention concerne un procédé enzymatique de préparation d'acides gras polyinsaturés à partir d'une huile riche en ces acides gras polyinsaturés, caractérisé par le fait que l'on hydrolyse l'huile par une lipase non stéréospécifique de Candida cylindracea en milieu émulsionné en présence de lécithine à titre d'agent émulsionnant.

Le procédé selon l'invention s'applique en particulier à l'hydrolyse des huiles riches en acides gras des séries n-3 et n-6, notamment en acide gammalinolénique, notamment les huiles de pépins de cassis, de bourrache et d'onagre.

Dans la mise en oeuvre du procédé, on conduit l'hydrolyse enzymatique complète des triglycérides des huiles riches en acides gras polyinsaturés en milieu émulsionné à température de 20 à 45° C, de préférence à la température ambiante, à la pression atmosphérique et à pH de 6 à 8. Ce sont des conditions ménageantes, qualitativement et économiquement intéressantes.

La réaction enzymatique a lieu à l'interface entre la phase organique et la phase aqueuse contenant l'enzyme et une solution aqueuse de tampon, de pH 6-8, proche de la neutralité. L'émulsion, de type huile-dans-l'eau est produite par agitation intense de l'huile en présence de la phase aqueuse et de l'émulsifiant pour produire une dispersion de l'huile sous forme de fines gouttelettes, par exemple de diamètre moyen environ 450 nm, dispersées dans la phase aqueuse et stabilisées par l'émuisifiant, une lécithine.

Une lécithine utilisable selon l'invention peut être, de préférence, choisie parmi les phospholipides suivants:
- Une lécithine contenant plus de 60 % en poids de matières insolubles dans l'acétone (ci-après AIM),
- Une lécithine déshuilée, contenant plus de 90 % en poids d'AIM,
- Une fraction de lécithine soluble dans l'alcool, contenant plus de 60 % en poids d'AIM, enrichie en phosphatidyl choline (PC),
- Une lécithine soluble dans l'alcool et déshuilée, contenant plus de 90 % en poids d'AIM,
- Une fraction de lécithine contenant plus de 50 % en poids de PC et
- Les lécithines ou leurs fractions précédentes modifiées par phospholipase A₂ d'origine pancréatique ou microbienne, dans lesquelles 10 à 90 % en poids de la PC est convertie en lyso-PC (LPC).

On peut préparer l'émulsion à l'avance en vue d'une utilisation ultérieure et par exemple la mettre dans des récipients que l'on stérilise.

La composition de l'émulsion est déterminée de manière à lui conférer une stabilité durable. Si l'on augmente la proportion d'huile au delà d'une certaine limite, par exemple au delà d'environ 20 % en poids, l'émulsion n'est plus assez homogène et le taux d'hydrolyse obtenu diminue. Une teneur en lécithine en dessous d'environ 1 % en poids conduit également à diminuer le taux d'hydrolyse. De préférence, la teneur en lécithine est 0,1 à 5 % en poids, avantageusement 0,3 à 0,8 % en poids et particulièrement environ 0,5 % en poids de la composition. De préférence, la teneur en lécithine est fixée pour correspondre à 1,5 à 4 % en poids, et particulièrement environ 2,5 % en poids de PC pure, basée sur la teneur en huile de l'émulsion.

La lipase non stéréospécifique est de Candida cylindracea. Les conditions optimales de réaction dépendent de la nature du substrat à hydrolyser et des propriétés intrinsèques des différentes lipases.

La réaction a lieu entre 20 et 45° C, de préférence à la température la plus basse, par exemple à environ 20° C, dans la mesure où l'hydrolyse complète est possible, ce qui permet en particulier de ménager les acides gras polyinsaturés particulièrement sensibles à la dégradation oxydative qui augmente avec la température.

Le pH n'influence pas le taux d'hydrolyse pour les valeurs entre 6 et 8. On utilise de préférence une solution tampon neutre d'un sel tel que, par exemple, un phosphate de pH environ 6,88, de manière à conserver des conditions de réaction conduisant à des résultats constants et reproductibles. Dans des conditions optimales décrites ci-dessus, l'hydrolyse complète peut être obtenue en 2 à 20 h.

Le taux d'hydrolyse dépend, dans les conditions opératoires définies précédemment de la concentration en enzyme utilisée, mais au delà d'une certaine limite, ce paramètre n'intervient plus, puisque la réaction enzymatique a lieu à l'interface entre l'huile et le milieu aqueux et que la quantité d'enzyme nécessaire passe par un maximum tributaire de la surface de l'interface disponible. On utilise de préférence une concentration de 10 à 100 mg/g d'huile.

Une fois la réaction terminée, la lipase peut de préférence être recyclée et réutilisée. Pour ce faire, on centrifuge l'émulsion à vitesse élevée pour la casser, puis on récupère la phase lipidique par extraction à l'aide d'un solvant, lavage à l'eau, séchage sur sulfate de sodium et évaporation du solvant. On conserve de préférence les acides gras à l'abri de la lumière, sous atmosphère inerte et à température négative (en échelle Celcius), de préférence à environ - 25° C. On récupère la lipase en solution dans la phase aqueuse avec le glycérol formé lors de l'hydrolyse en la concentrant, par exemple par ultrafiltration. La lipase peut alors être recyclée.

Le mélange d'acides gras obtenu peut être utilisé comme matière première d'un procédé d'enrichissement en acides gras polyinsaturés spécifiques ou pour la préparation de triglycérides structurés dans les quels les acides gras ainsi que leurs positions dans la molécule de glycérol sont spécifiques. Les produits obtenus peuvent être à leur tour utilisés dans des compositions nutritionnelles, cosmétiques et pharmaceutiques.

Les exemples ci-après illustrent l'invention. Dans ceux-ci, les pourcentages et parties sont en poids, sauf indication contraire.

### Exemples 1-7

On prépare une émulsion huile-dans-l'eau contenant 20 % d'huile de pépins de cassis et 1,2 % de lécithine de soja (Asol 100 (R), Lucas Meyer) mise en solution dans 78,8 % d'une solution aqueuse de tampon phosphate 0,025 M de pH 6,88 et en effectuant 5 passages dans un microfluidiseur (110T, Microfluidics Corporation, Newton), ce qui conduit à un diamètre moyen des gouttelettes d'huile de 450 nm environ.

On solubilise la lipase dans le tampon phosphate, puis on la centrifuge à 4000 g pendant 20 min. pour éliminer les résidus insolubles. Le surnageant est utilisé pour les expériences. 10 ml de l'émulsion précédente (contenant 2 g d'huile de pépins de cassis) sont placés dans un Erlenmeyer bouché de 25 ml, dans un bain thermostaté à la température choisie sous agitation magnétique à 250 tour/min. à laquelle on ajoute la solution enzymatique correspondant à 0,2 g de lipase.

Après réaction, on centrifuge le milieu à 4000 g pour casser l'émulsion et on récupère la phase lipidique par extraction à l'éther. On lave l'extrait à l'eau, on le sèche sur sulfate de sodium, puis on élimine le solvant par évaporation. On conserve les acides gras obtenus à -25° C à l'abri de la lumière et sous azote.

On récupère la lipase en solution dans la phase aqueuse ainsi que le glycérol formé par ultrafiltration (module YM10, seuil de coupure 10000, Amicon, Denver, USA), ce qui donne une solution concentrée de lipase qui peut être réutilisée.

Le taux d'hydrolyse, correspondant au pourcentage d'acides gras libérés au cours de la réaction a été déterminé par titration acide-base à l'aide d'un titroprocesseur 692 de Metrohm. L'échantillon à analyser, dissout dans 25 ml d'un mélange équivolumique d'éthanol et d'éther éthylique a été titré par une solution alcoolique de KOH de concentration 0,1 N.

Les conditions de l'hydrolyse sont indiquées dans le tableau I ci-après:

**Tableau I**

| Exemple | Lipase de Candida cylindracea | Température, ° C | Durée, h | % Hydrolyse |
|---|---|---|---|---|
| 1 | Type B, Biocatalysts Ltd., Cardiff, Angleterre | 37 | 4 | 99,9 |
| 2 | Type VII, Sigma Chemical Co., St. Louis, USA | 20 | 20 | 91,6 |
| 3 | Lot 189, Biogenzia Lemania SA, Lausanne, Suisse | 37 | 20 | 96 |
| 4 | Type OF, Meito Sangyo Co. Ltd. Tokyo, Japon | 20 | 8 | 99,2 |
| 5 | Type OF, Meito Sangyo Co. Ltd. Tokyo, Japon | 30 | 8 | 96,3 |
| 6 | Type MY, Meito Sangyo Co. Ltd. Tokyo, Japon | 37 | 20 | 91,1 |
| 7 | Type F5, Enzymatix, Cambridge, Angleterre | 37 | 20 | 97,6 |

A titre de comparaison, l'hydrolyse en milieu biphasique en utilisant les enzymes et les conditions de température et de durée des exemples 1 et 4 a conduit aux résultats suivants:

| Lipase de l'exemple | % Hydrolyse en milieu biphasique |
|---|---|
| 1 | 79 |
| 4 | 81,1 |

### Exemples 8-13

En variant la concentration en enzyme, les proportions d'huile et de lécithine et le nombre de cycles successifs avec la lipase recyclée tout en utilisant les mêmes autres conditions que dans l'exemple 4, on a obtenu les taux d'hydrolyse indiqués dans le tableau II ci-après:

**Tableau II**

| Exemple | Composition de l'émulsion (%) | Concentration en enzyme, mg/g d'huile | Nombre de cycles d'hydrolyse de 20 h | % Hydrolyse |
|---|---|---|---|---|
| 8 | huile, 30 | - | - | 78 |
| | lécithine, 1,2 | | | |
| | tampon, 68,8 | | | |
| 9 | huile, 20 | - | - | 89 |
| | lécithine, 1 | | | |
| | tampon, 79 | | | |
| 10 | - | 20 | - | 88,6 |
| 11 | - | 50 | - | 92,7 |
| 12 | - | - | 2 | 96 |
| 13 | - | - | 3 | 94,6 |
| -: Mêmes conditions que dans l'exemple 4. | | | | |

### Exemple 14

On a évalué la qualité et la stabilité des acides gras obtenus par la voie enzymatique en comparaison avec celle obtenue par saponification chimique de l'huile de pépins de cassis dans les conditions suivantes:
On mélange 100 g d'huile de pépins de cassis avec 213 g d'une solution alcaline composée de 47,7 % d'eau, de 14,3 % d'hydroxyde de sodium, de 37,8 % d'éthanol et de 0,15 % d'éthylènediaminetétraacétatedisodique et on porte le mélange à reflux à 80-85°. Cependant 120 min. Après addition de 40 g d'eau, puis de 80 ml d'acide chlorhydrique fumant, on remue vivement le mélange pendant 60 min. On extrait les acides gras formés à l'hexane, on lave la phase organique à l'eau, on la sèche sur sulfate de sodium et on élimine le solvant par évaporation.

Une analyse des acides gras des deux hydrolysats sous forme de leurs esters méthyliques par chromatographie en phase gazeuse a montré la parfaite concordance de leur composition, ce qui permet d'affirmer que la lécithine de soja n'est pas hydrolysée.

L'analyse par spectroscopie UV des acides gras en solution dans l'isooctane a montré que les valeurs des maxima d'adsorption des acides gras obtenus par hydrolyse enzymatique sont moins élevées que celles des acides gras obtenus chimiquement. De plus, les valeurs des coefficients d'extinction molaire, calculées pour plusieurs longueurs d'onde et pour les deux types d'acide gras, sont plus élevées dans le cas des acides gras obtenus chimiquement que dans celui des acides gras obtenus enzymatiquement.

Enfin, la détermination de l'indice de peroxyde des acides gras permet de comparer les deux méthodes d'hydrolyse et la qualité des acides gras obtenus. Les valeurs trouvées par transformation des ions ferriques en thiocyanate ferrique de couleur rouge mesurée à la longueur d'onde de 510 nm sur un spectrophotomètre Hewlett Packard 845A permettent de déterminer l'indice de peroxyde exprimé en meq d'O₂/kg. Les résultats sont les suivants:

| | |
|---|---|
| Acides gras obtenus par saponification | 7,9 |
| Acides gras obtenus par voie enzymatique | 1,4 |

Les résultats analytiques précédents confirment que la réaction d'hydrolyse biocatalysée conduit à un mélange d'acides gras de qualité sensiblement meilleure que l'hydrolyse chimique.

## Revendications

1. Procédé enzymatique de préparation d'acides gras polyinsaturés à partir d'une huile riche en ces acides gras polyinsaturés, caractérisé par le fait que l'on hydrolyse l'huile par une lipase non stéréospécifique de Candida cylindracea en milieu émulsionné en présence de lécithine à titre d'agent émulsionnant.

2. Procédé selon la revendication 1, caractérisé par le fait que l'émulsion est du type huile-dans-l'eau et contient jusqu'à 30 % en poids d'huile et 0,1 à 5 %, et notamment 0,3 à 0,8 % en poids de lécithine.

3. Procédé selon la revendication 1, caractérisé par le fait que la réaction a lieu à la température ambiante.

4. Procédé selon la revendication 1, caractérisé par le fait que la phase aqueuse contient un sel tampon maintenant le pH autour de la neutralité.

5. Procédé selon la revendication 1, caractérisé par le fait que l'on utilise une quantité d'enzyme correspondant à 10 à 100 mg/g d'huile.

6. Procédé selon la revendication 1, caractérisé par le fait que la réaction a lieu pendant 2 à 20 h.

7. Procédé selon la revendication 1, caractérisé par le fait que, une fois la réaction terminée, on centrifuge l'émulsion à vitesse élevée pour la casser, puis on récupère la phase lipidique par extraction à l'aide d'un solvant, lavage à l'eau, séchage et évaporation du solvant et on conserve les acides gras obtenus à l'abri de la lumière, sous atmosphère inerte et à température négative en attente d'utilisation.

8. Procédé selon la revendication 1, caractérisé par le fait que l'on récupère la lipase en solution dans la phase aqueuse avec le glycérol formé lors de l'hydrolyse en la concentrant par ultrafiltration et on la recycle pour la réutiliser dans une nouvelle hydrolyse.

9. Procédé selon la revendication 1, caractérisé par le fait que l'huile mise en oeuvre est une huile riche en acides gras des séries n-3 et n-6, notamment en acide gammalinolénique, notamment choisie parmi l'huile de pépins de cassis, l'huile de bourrache et l'huile d'onagre.

10. Mélange d'acides gras obtenu par la mise en oeuvre du procédé selon l'une des revendications 1 à 9.
